(19) Europäisches Patentamt
European Patent Office
Office européen des brevets

(11) **EP 4 223 271 A1**

(12) **EUROPEAN PATENT APPLICATION**
published in accordance with Art. 153(4) EPC

(43) Date of publication:
**09.08.2023 Bulletin 2023/32**

(21) Application number: **21875891.0**

(22) Date of filing: **01.10.2021**

(51) International Patent Classification (IPC):
*A61K 6/78* (2020.01)     *A61C 13/00* (2006.01)
*A61C 13/083* (2006.01)     *A61K 6/818* (2020.01)
*A61K 6/822* (2020.01)

(52) Cooperative Patent Classification (CPC):
**A61C 13/00; A61C 13/083; A61K 6/78;
A61K 6/818; A61K 6/822**

(86) International application number:
**PCT/JP2021/036505**

(87) International publication number:
**WO 2022/071595 (07.04.2022 Gazette 2022/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.10.2020 JP 2020168076**

(71) Applicant: **Kuraray Noritake Dental Inc.
Kurashiki-shi, Okayama 710-0801 (JP)**

(72) Inventors:
• **SAKAMOTO Hiroyuki**
  **Miyoshi-shi, Aichi 470-0293 (JP)**
• **KASHIKI Nobusuke**
  **Miyoshi-shi, Aichi 470-0293 (JP)**
• **KATO Shinichiro**
  **Miyoshi-shi, Aichi 470-0293 (JP)**

(74) Representative: **D Young & Co LLP
120 Holborn
London EC1N 2DY (GB)**

(54) **DENTAL CERAMIC COLORING SOLUTION**

(57)     The present invention provides a dental ceramic coloring solution with which the pink shade needed for dental use can be imparted while reducing a decrease in the strength of a dental ceramic. The present invention relates to a coloring solution for coloring a dental ceramic, comprising a coloring component and a solvent, wherein the coloring component comprises an Er component and a Co component.

EP 4 223 271 A1

Processed by Luminess, 75001 PARIS (FR)

**Description**

TECHNICAL FIELD

The present invention relates to a coloring solution for dental ceramics. Specifically, the invention relates to a dental ceramic coloring solution suited for use in the fabrication of dental prostheses machined with a dental CAD/CAM system, for example, such as inlays, onlays, veneers, crowns, bridges, abutment teeth, dental posts, dentures, denture bases, and implant parts (fixtures, abutments).

BACKGROUND ART

Traditionally, metal has been used for a range of dental products, including, for example, dental prostheses (such as veneer crowns, dental caps, crowns, and post crowns), orthodontic products, and products for dental implants. However, metals lack aesthetic quality because of the colors that are distinctively different from the color of natural teeth, and can cause allergic reaction when released from these products. These issues involving the use of metal have been addressed by dental products that use a ceramic material such as aluminum oxide (alumina) or zirconium oxide (zirconia) as an alternative material of metal. Particularly, zirconia excels in strength, and has relatively good aesthetics, and this, combined in particular with the currently declining price of zirconia, has created a high demand for this material.

In recent years, there has been increasing use of a CAD/CAM system that makes use of a computer for designing and a milling machine for machining to form the final shape of a dental prosthesis, or to fabricate a prosthesis for large implants. For aesthetic values, the CAD/CAM system typically uses zirconia as a material of a mill blank to be milled. Particularly, recent years have seen increased use of zirconia that satisfies aesthetic requirements by reproducing the color of natural teeth with different shades of color vertically arranged in a thickness direction. For shades that are difficult to reproduce, a ceramic that has been worked into a shape of a dental prosthesis is colored by coating dental porcelain over a ceramic surface to satisfy high aesthetic requirements.

However, color coating with dental porcelain requires expertise and great technique. Specifically, high skills are required for coloring with dental porcelain because dental porcelain needs to be built up to develop colors with high aesthetics while reproducing the shape and structure of natural teeth with precision.

In a common technique used to circumvent the issues involved in the use of dental porcelain, a method is available that colors a dental prosthesis by applying a coloring solution to a dental ceramic to achieve even better aesthetics, instead of the skillful coloring with dental porcelain.

For example, Patent Literature 1 discloses a coloring solution for coloring a dental ceramic article. The coloring solution comprises a coloring agent comprising rare earth element metals or ions, and transition metals or ions. Erbium is given as an example of the rare earth elements.

CITATION LIST

Patent Literature

Patent Literature 1: JP 2010-534245 T

SUMMARY OF INVENTION

Technical Problem

There are circumstances where a dental ceramic needs to be colored pink. A prosthesis including a gingival part is an example of a dental material that requires pink coloring. As an example, a prosthesis including a gingival part may be an implant's upper structure used beneath the gingival margin, or a large dental prosthesis called ALL ON 4 (a procedure with balanced placement of four implants in the bone), which enables prosthetic reproduction of teeth, including the gingival area. When used as a coloring solution for zirconia in such applications, the coloring solution of Patent Literature 1 must contain a large amount of erbium (e.g., about 15 mass%) to provide the pink color needed in these applications. However, because erbium also acts as a zirconia stabilizer, containing a large amount of erbium in the coloring solution causes a decrease in the strength and other properties of zirconia. That is, it has not been possible to satisfy both pink coloring and strength in dental ceramics in dental applications requiring pink coloring.

It is accordingly an object of the present invention to provide a dental ceramic coloring solution with which the pink shade needed for dental use can be imparted while reducing a decrease in the strength of a dental ceramic.

Solution to Problem

The present inventors conducted intensive studies to find a solution to the foregoing problem, and found that a coloring solution capable of developing the pink shade needed for dental use can be obtained by containing a Co component in addition to an Er component, even when the content of the Er component is reduced. The present invention was completed after further studies.

Specifically, the present invention includes the following.

[1] A coloring solution for coloring a dental ceramic, comprising a coloring component and a solvent, wherein the coloring component comprises an Er component and a Co component.

[2] The coloring solution according to [1], wherein a dental ceramic after coloring and sintering has an a* of 4.5 to 15.0, and a b* of -5.0 to 10 in (L*,a*,b*) according to L*a*b*color system.

[3] The coloring solution according to [1] or [2], wherein L* in (L*,a*,b*) according to L*a*b*color system is 65 to 95.

[4] The coloring solution according to any one of [1] to [3], wherein the coloring component further comprises an Al component.

[5] The coloring solution according to any one of [1] to [4], wherein the Er component is an ion or a complex.

[6] The coloring solution according to any one of [1] to [5], wherein the Er component is a component derived from at least one selected from the group consisting of erbium chloride hydrate, erbium perchlorate hydrate, erbium nitrate hydrate, erbium oxalate hydrate, and erbium acetate hydrate.

[7] The coloring solution according to any one of [1] to [6], wherein the Co component is an ion or a complex.

[8] The coloring solution according to any one of [1] to [6], wherein the Co component is a component derived from at least one selected from the group consisting of cobalt(II) chloride hydrate, cobalt(II) perchlorate hydrate, cobalt(II) fluoride hydrate, cobalt(II) nitrate hydrate, cobalt(II) oxalate hydrate, and cobalt(II) acetate hydrate.

[9] The coloring solution according to any one of [1] to [8], wherein the content of the Er component is 110 to 310 mmol/L in terms of an Er ion.

[10] The coloring solution according to any one of [1] to [9], wherein the content of the Co component is 0.0340 to 1.70 mmol/L in terms of a Co ion.

[11] The coloring solution according to any one of [1] to [10], wherein the solvent comprises water and/or an organic solvent.

[12] The coloring solution according to [11], wherein the organic solvent comprises at least one selected from the group consisting of an alcohol, a glycol, a triol, and a ketone.

[13] The coloring solution according to any one of [1] to [12], wherein the dental ceramic comprises zirconia as a main component.

[14] The coloring solution according to [13], wherein the dental ceramic further comprises yttria.

[15] The coloring solution according to [14], wherein the content of the yttria is 1.5 to 10 mol% relative to a total mole of the zirconia and the yttria.

[16] A dental ceramic supporting an Er component and a Co component on a surface of the dental ceramic.

[17] The dental ceramic according to [16], which additionally supports an Al component on a surface of the dental ceramic.

[18] The dental ceramic according to [16] or [17], wherein the Er component is an ion or a complex.

[19] The dental ceramic according to any one of [16] to [18], wherein the Co component is an ion or a complex.

[20] The dental ceramic according to any one of [16] to [19], wherein the dental ceramic comprises zirconia as a main component.

[21] The dental ceramic according to [20], wherein the dental ceramic further comprises yttria.

[22] The dental ceramic according to [21], wherein the content of the yttria is 1.5 to 10 mol% relative to a total mole of the zirconia and the yttria.

Advantageous Effects of Invention

According to the present invention, a dental ceramic coloring solution can be provided with which the pink shade needed for dental use can be imparted while reducing a decrease in the strength of a dental ceramic.

DESCRIPTION OF EMBODIMENTS

The present invention is a coloring solution for coloring a dental ceramic, comprising a coloring component and a solvent, wherein the coloring component comprises an Er component and a Co component.

Er Component

The Er component contained in a coloring component of the present invention is described first. The Er component comprises an Er ion or an Er complex, and is contained to give a pink color to zirconia.

Ions or complexes of Er may be added in the coloring solution in the form of salts containing cations and anions of Er, or in the form of complexes containing Er and its ligands. Examples of the anions or ligands include $OAc^-$, $NO_3^-$, $NO_2^-$, $CO_3^{2-}$, $HCO_3^-$, $ONC^-$, $SCN^-$, $SO_4^{2-}$, $SO_3^{2-}$; glutarate, lactate, gluconate, propionate, butyrate, glucuronate, benzoate, phenolate, halide anions (fluorides, chlorides, bromides), and acetate. Ac means acetyl group.

Specific examples of compounds added to contain the foregoing ions or complexes in a coloring solution of the present invention include trivalent erbium compounds such as tris(acetylacetonate)erbium hydrate, erbium chloride hydrate (erbium chloride hexahydrate), erbium perchlorate hydrate, erbium acetate hydrate (erbium acetate tetrahydrate), erbium oxide, erbium oxalate hydrate, erbium nitrate hydrate, erbium stearate, erbium fluoride, erbium tetraboride, erbium iodide hydrate, erbium sulfide, and erbium sulfate hydrate. In view of solubility and chromogenicity, preferred are erbium chloride hydrate, erbium perchlorate hydrate, erbium nitrate hydrate, erbium oxalate hydrate, and erbium acetate hydrate. The Er component may be used alone, or two or more thereof may be used in an appropriate combination.

The content of the Er component in the coloring solution is preferably 110 to 310 mmol/L, more preferably 120 to 300 mmol/L, even more preferably 130 to 290 mmol/L in terms of an Er ion in the whole solution. When the content of the Er component is less than 110 mmol/L, it may not be possible to satisfy high aesthetic requirements because of insufficient development of a pink color. When contained in an amount of more than 310 mmol/L, the Er component may act as a zirconia stabilizer, and the strength and other properties of zirconia may decrease. The content of the Er component can be measured using a technique, for example, such as inductively coupled plasma (ICP) emission spectral analysis, or X-ray fluorescence analysis. In the present specification, the contents of metallic components can be measured by using these methods.

Co Component

The following describes the Co component contained in a coloring solution of the present invention. The Co component comprises a Co ion or a Co complex, and is contained to give a pink color without decreasing the strength and other properties of zirconia.

By applying a dental ceramic coloring solution containing a Co component, the desired pink shade can be imparted to a dental ceramic without decreasing its strength. Though the reason for this remains unclear, the present inventors have proposed the following explanation. When the dental ceramic is zirconia, adding $Co^{2+}$ ions of lower valency than $Zr^{4+}$ ions introduces oxygen ion vacancy in zirconia crystal structure, and decreases the actual ion radius of $O^{2-}$ ions. Presumably, the resulting increase in the radius ratio of $O^{2-}$ ions and $Zr^{4+}$ ions stabilizes the fluorite structure, and reduces a decrease of zirconia strength.

Ions or complexes of Co may be added in the coloring solution in the form of salts containing cations and anions of Co, or in the form of complexes containing Co and its ligands. Examples of the anions or ligands include $OAc^-$, $NO_3^-$, $NO_2^-$, $CO_3^{2-}$, $HCO_3^-$, $ONC^-$, $SCN^-$, $SO_4^{2-}$; $SO_3^{2-}$; glutarate, lactate, gluconate, propionate, butyrate, glucuronate, benzoate, phenolate, halide anions (fluorides, chlorides, bromides), and acetate.

Specific examples of compounds added to contain the foregoing ions or complexes in a coloring solution of the present invention include bis(acetylacetonate)diaqua cobalt(II), tris(acetylacetonate)cobalt(III), cobalt(II) amidosulfate hydrate, cobalt(II) benzoate, cis-tetraamminedichlorocobalt(III) chloride, pentaamminechlorocobalt(III) chloride, hexaamminecobalt(III) chloride, hexaamminecobalt(III) nitrate, ammonium diamminetetranitrocobaltate(III), triammine trinitro cobalt(III), tetraamminedinitrocobalt(III) chloride, potassium diamminetetranitrocobaltate(III), cobalt(II) chloride hydrate (for example, cobalt(II) chloride hexahydrate), cobalt(II) chloride, cobalt(III) chloride, cobalt(II) octanoate, cobalt(II) oleate, cobalt(II) perchlorate hydrate (cobalt(II) perchlorate hexahydrate), cobalt(II) fluoride hydrate (cobalt(II) fluoride dihydrate, cobalt(II) fluoride trihydrate, cobalt(II) fluoride tetrahydrate), dicobalt octacarbonyl ($Co_2(CO)_8$), cobalt(II) formate hydrate, cobalt(II) citrate hydrate, cobalt disilicide, cobalt(II) acetate, cobalt(II) acetate hydrate (cobalt(II) acetate tetrahydrate), cobalt(II) oxide, cobalt(III) oxide, tricobalt tetroxide ($Co_3(CO)_4$), potassium hexacyanocobaltate(III), cobalt(II) bromide, cobalt(II) bromide hydrate, cobalt(II) oxalate hydrate (for example, such as cobalt(II) oxalate dihydrate, and cobalt(II) oxalate tetrahydrate), cobalt resinate, cobalt(II) nitrate hydrate (cobalt(II) nitrate trihydrate, cobalt(II) nitrate hexahydrate), cobalt(II) metazirconate, cobalt(II) hydroxide, cobalt(III) hydroxide, cobalt(II) stearate, cobalt(II) selenate, cobalt(II) selenate hexahydrate, cobalt(II) tungstate tetrahydrate, cobalt(II) carbonate hydroxide, ammonium tetrakis(thiocyanate)cobaltate(II) hydrate, cobalt(II) thiocyanate, cobalt(II) metatitanate, potassium hexanitrocobaltate(III), sodium hexanitrocobaltate(III), hexaamminecobalt(III) sulfate, cobalt boride, cobalt(II) molybdate hydrate, cobalt(II) iodide hydrate, cobalt(II) laurate, cobalt(II) sulfide, cobalt(II) sulfate hydrate, cobalt(II) phosphide, and cobalt(II) phosphate hydrate. In view of solubility and chromogenicity, preferred are cobalt(II) chloride hydrate, cobalt(II) perchlorate hydrate, cobalt(II) fluoride hydrate, cobalt(II) nitrate hydrate, cobalt(II) oxalate hydrate, and cobalt(II) acetate hydrate. These Co components may be used alone, or two or more thereof may be used in an appropriate combination.

The content of the Co component in the coloring solution is preferably 0.0340 to 1.70 mmol/L, more preferably 0.0380 to 1.50 mmol/L, even more preferably 0.0420 to 1.30 mmol/L in terms of a Co ion in the whole solution. When the content of the Co component is less than 0.0340 mmol/L, the Er component must be contained in an amount of more than 310 mmol/L to impart a pink color, and the strength and other properties of zirconia may decrease. When the Co component is added in an amount of more than 1.70 mmol/L, it may not be possible to give a pink color because of an increased blue tint.

Preferably, a coloring solution of the present invention further comprises an Al component. The Al component comprises an Al ion or an Al complex, and can increase the red tint. The Al component may improve the strength of zirconia.

By applying a dental ceramic coloring solution containing an Al component, it is possible to increase the red tint, and improve the strength of zirconia. Though the reason for this remains unclear, the present inventors have proposed the following explanation. The increased red tint is probably because of the presence of the corundum crystal structure of Al with the Co component, producing a reddish purple color, and increasing the overall red tint. The improved zirconia strength is probably due to the Al component being dispersed between zirconia crystals, and developing its high toughness characteristics on the binding force between crystals by improving the intercrystalline binding force with an increased specific surface area of bonding between crystals.

Ions or complexes of Al may be added in the coloring solution in the form of salts containing cations and anions of Al, or in the form of complexes containing Al and its ligands. Examples of the anions or ligands include $OAc^-$, $NO_3^-$, $NO_2^-$, $CO_3^{2-}$, $HCO_3^-$, $ONC^-$, $SCN^-$, $SO_4^{2-}$, $SO_3^{2-}$; glutarate, lactate, gluconate, propionate, butyrate, glucuronate, benzoate, phenolate, halide anions (fluorides, chlorides, bromides), and acetate.

Specific examples of compounds added to contain the foregoing ions or complexes in a coloring solution of the present invention include aluminum ethoxide, aluminum butoxide, aluminum propoxide, barium aluminate, magnesium aluminate, lithium aluminate, aluminum benzoate, aluminum chloride hydrate (aluminum chloride hexahydrate), aluminum oleate, aluminum perchlorate hydrate (such as aluminum perchlorate trihydrate, and aluminum perchlorate hexahydrate), aluminum citrate hydrate (aluminum citrate monohydrate), aluminum gluconate, lithium tetrachloroaluminate, lithium aluminum chloride, aluminum oxide, aluminum selenate hydrate, aluminum oxalate hydrate, aluminum tartrate hydrate, aluminum metazirconate, aluminum octanoate hydroxide, aluminum stearate, aluminum titanate, aluminum lactate, aluminum palmitate, lithium tetrahydridoaluminate, lithium aluminum hydride, aluminum iodide, aluminum laurate, aluminum butyrate, aluminum nitrate hydrate (such as aluminum nitrate nonahydrate), aluminum sulfide, and aluminum cesium sulfate hydrate. In view of solubility, preferred are aluminum chloride hydrate, aluminum perchlorate hydrate, aluminum oxalate hydrate, and aluminum nitrate hydrate. These Al components may be used alone, or two or more thereof may be used in an appropriate combination.

The content of the Al component in the coloring solution is preferably 0.100 to 40.0 mmol/L, more preferably 0.200 to 30.0 mmol/L, even more preferably 0.400 to 25.0 mmol/L in the whole solution. When the Al component is added in an amount of less than 0.100 mmol/L, it may not be possible to obtain a sufficient red tint, or produce the zirconia strength improving effect. When added in an amount of more than 40.0 mmol/L, the Al component may cause decreased aesthetics.

A coloring solution of the present invention may comprise other metallic components, provided that it does not hinder the effects of the present invention. For example, the strength of zirconia after the application of the coloring solution may improve when the coloring solution comprises a Zr component.

The Zr component comprises a Zr ion or a Zr complex. Ions or complexes of Zr may be added in the coloring solution in the form of salts containing cations and anions of Zr, or in the form of complexes containing Zr and its ligands. Examples of the anions or ligands include $OAc^-$, $NO_3^-$, $NO_2^-$, $CO_3^{2-}$, $HCO_3^-$, $ONC^-$, $SCN^-$, $SO_4^{2-}$; $SO_3^{2-}$; glutarate, lactate, gluconate, propionate, butyrate, glucuronate, benzoate, phenolate, halide anions (fluorides, chlorides, bromides), and acetate.

Specific examples of compounds added to contain the foregoing ions or complexes in a coloring solution of the present invention include zirconium(IV) chloride oxide hydrate, zirconium(IV) sulfide, zirconium(IV) tetrakis(acetylacetonate), zirconium(IV) chloride, zirconium(IV) octanoate, zirconium(IV) oleate oxide, dichlorobis(η5-cyclopentadienyl)zirconium(IV), zirconium(IV) acetate oxide, zirconium(IV) oxide hydrate, zirconium(IV) stearate oxide, zirconium(IV) nitrate oxide hydrate, zirconium(IV) n-butoxide, zirconium(IV) carbide, dizirconium(IV) carbonate trioxide hydrate ($ZrOCO_3 \cdot ZrO_2 \cdot nH_2O$), ammonium hexafluorozirconate(IV), zirconium(IV) iodide, zirconium(IV) laurate oxide ($Zr(C_{11}H_{23}COO)_2O$), zirconium(IV) sulfate hydrate, and zirconium(IV) dihydrogen phosphate oxide. In view of the ability to further improve the dental ceramic strength, preferred are zirconium(IV) chloride oxide hydrate, zirconium(IV) chloride, zirconium(IV) acetate oxide, zirconium(IV) oxide hydrate, and zirconium(IV) nitrate oxide hydrate, more preferably zirconium(IV) chloride oxide hydrate. The Zr component may be used alone, or two or more thereof may be used in an appropriate combination.

In view of improving the strength of a dental ceramic, the content of the Zr component in the coloring solution is preferably 0.0650 to 0.900 mol/L, more preferably 0.0800 to 0.850 mol/L, even more preferably 0.0900 to 0.800 mol/L in the whole solution. When the content of the Zr component is 0.0650 mol/L or more, it is possible to produce the effect to improve the strength of a dental ceramic. When the content of the Zr component is 0.900 mol/L or less, the strength can improve without decreasing the aesthetics of the dental ceramic.

Preferably, a coloring solution of the present invention comprises water and/or an organic solvent as solvent. Water and/or an organic solvent allow the coloring component and the Zr component to dissolve, and improve the penetration of the coloring solution into a dental ceramic. The solvent content in the coloring solution is preferably 45 to 99 mass%, more preferably 60 to 98.5 mass%, even more preferably 75 to 98 mass%.

It is required that water be essentially free of impurities that are detrimental to the effects of the present invention. Preferably, water is purified water, distilled water, ion-exchange water, or deionized water. The content of water in the coloring solution is preferably 45 to 99 mass%, more preferably 60 to 98.5 mass%, even more preferably 75 to 98 mass%.

The organic solvent may be any solvent that can dissolve the cations, and is preferably an alcohol, a glycol, a triol, or a ketone, or a mixture selected from combinations of these. Specific examples include:

alcohols such as methanol, ethanol, 1-propanol, 2-propanol, isopropanol, 1-butanol, 2-butanol, 1-heptanol, 2-heptanol, 3-heptanol, 1-hexanol, 2-hexanol, 3-hexanol, 2-methyl-1-pentanol, 3-methyl-1-pentanol, 4-methyl-1-pentanol, 2-methyl-2-pentanol, 2-methyl-3-pentanol, 2,2-dimethyl-1-butanol, 2-ethyl-1-butanol, ethylene glycol monomethyl ether, ethylene glycol monoethyl ether, ethylene glycol monobenzyl ether, propylene glycol monomethyl ether, propylene glycol monoethyl ether, ethylene glycol monobutyl ether, diethylene glycol monomethyl ether, diethylene glycol monoethyl ether, diethylene glycol monobenzyl ether, propylene glycol monopropyl ether, tripropylene glycol monomethyl ether, diethylene glycol monobutyl ether, triethylene glycol monomethyl ether, benzyl alcohol, 2-(benzyloxy)ethanol, 3-(benzyloxy)-1-propanol, 2-(benzyloxy)-1-butanol, and 5-(benzyloxy)-1-pentanol;
diols such as 1,2-ethanediol, 1,2-propanediol, 1,3-propanediol, 1,2-butanediol, 1,3-butanediol, 1,4-butanediol, 2,3-butanediol, 1,2-pentanediol, 1,5-pentanediol, 2,4-pentanediol, 1,2-hexanediol, 2,5-hexanediol, ethylene glycol, diethylene glycol, triethylene glycol, tetraethylene glycol, polyethylene glycol (a molecular weight of 200 to 600), propylene glycol, dipropylene glycol, polypropylene glycol, 1-methyl-1,3-propanediol, 2-methyl-1,3-propanediol, 2-methyl-1,4-butanediol, 3-methyl-1,3-butanediol, 2-methyl-2,4-pentanediol, 3-methyl-1,5-pentanediol, 2,4-diethyl-1,5-pentanediol, and 2-ethyl-1,3-hexanediol;
triols such as glycerin, 1,2,4-butanetriol, 1,2,3-butanetriol, and 1,2,6-hexanetriol; and
ketones such as acetone, 2-butanone, 2-pentanone, isobutyl ketone, diisobutyl ketone, and cyclohexanone.

These organic solvents may be used alone, or two or more thereof may be used in an appropriate combination. The organic solvent may be used as a thickener (described later) to adjust viscosity.

The organic solvent content in the coloring solution is preferably 45 to 99 mass%, more preferably 60 to 98.5 mass%, even more preferably 75 to 98 mass%.

The coloring solution may comprise a complexing agent to such an extent that it does not hinder the effects of the present invention. Adding a complexing agent may be beneficial in improving the storage stability of the coloring solution, accelerating the dissolution process of the salts added to the coloring solution, and/or increasing the amount of salts that can dissolve in the coloring solution.

The complexing agent can typically form complexes with the metal ions present in the coloring solution. The complexes formed must be soluble in solvent. For example, the complexing agent may be used in at least a stoichiometric proportion in relation to the molar quantity of the ions contained in the coloring solution. Desirable results can be obtained when the mole ratio of the complexing agent is about 1 or about 2, or about 3 or more relative to the cations in the coloring solution.

Examples of the complexing agent include N,N-di(2-hydroxyethyl)glycine, acetylacetonate, crown ether, cryptands, ethylenediaminetriacetate and salts thereof, ethylenediaminetetraacetate and salts thereof, nitrilotriacetate and salts thereof, citric acid and salts thereof, triethylenetetraamine, porphin, polyacrylate, polyaspartate, acidic peptides, phthalocyanine, salicylate, glycinate, lactate, propylenediamine, ascorbate, oxalic acid and salts thereof, and a mixture of these. The complexing agent may be used alone, or two or more thereof may be used in an appropriate combination.

The content of the complexing agent in the coloring solution is not particularly limited, as long as the present invention can exhibit its effects. For example, it is preferable to contain the complexing agent in an amount sufficient to dissolve the cations in the solution, or to prevent precipitation of the cations. Specifically, the content of the complexing agent in the coloring solution is preferably 0.01 mass% or more, more preferably 0.05 mass% or more, even more preferably 0.10 mass% or more. There is no specific upper limit for the content of the complexing agent. However, the content of the complexing agent is preferably 50 mass% or less, more preferably 20 mass% or less, even more preferably 10 mass% or less. The complexing agent may fail to fully dissolve the cations when used in excessively small amounts, whereas the excess portion of the complexing agent itself may remain without dissolving when the complexing agent is used in excessively large amounts.

A coloring solution of the present invention has a pH of preferably 0 to 9, more preferably 1 to 7, even more preferably 2 to 6. The cations may start precipitating from the solution when the pH falls outside of these ranges. For example, the pH is preferably 0 to 9 when the coloring solution is an aqueous solution. The pH is preferably 0 to 6 when the coloring solution does not contain a complexing agent, and is preferably 3 to 9 when the coloring solution contains a complexing agent. The pH can be measured with a commercially available pH meter (for example, a compact pH meter LAQUAtwin manufactured by Horiba Ltd.).

Preferably, a coloring solution of the present invention has an appropriate viscosity so that the solution, in addition to being applied to a ceramic surface in necessary amounts, can move into the pores of a ceramic unfired body (also referred to as "molded body" in the present specification) or a ceramic pre-sintered body. The appropriate viscosity is, for example, preferably 0.1 to 10,000 mPa, more preferably 0.5 to 6,000 mPa, even more preferably 1 to 3,000 mPa at 20°C. When the viscosity is too high, it may not be possible to contain the coloring solution in the pores of a ceramic unfired body or a ceramic pre-sintered body. The viscosity can be measured at 25°C with a Brookfield viscometer, though the method of viscosity measurement is not particularly limited.

In order to achieve the appropriate viscosity, a coloring solution of the present invention may comprise a thickener to such an extent that it does not hinder the effects of the present invention.

The thickener may be selected from the above organic solvents to adjust viscosity, or may be selected from the following thickeners. Examples of thickeners other than the organic solvents include:

polysaccharide compounds such as methyl cellulose, carboxycellulose, hydroxyethyl cellulose, xanthan gum, guar gum, carrageenan, tamarind seed gum, and pectin;
sugar alcohol compounds such as sorbitol, erythritol, xylitol, and trehalose;
synthetic polyol compounds such as diglycerin, triglycerin, polyglycerin, and polyvinyl alcohol; and
solid organic compounds such as sodium polyacrylate, ammonium polyacrylate, polyethylene oxide, polyethylene glycol (a molecular weight of 1,000 or more), polyvinylpyrrolidone, calcium stearate, magnesium stearate, zinc stearate, aluminum stearate, polyethylene glycol monostearate, 12-hydroxystearic acid, stearamide, oleamide, and ethylenebisoleamide. The thickener may be used alone, or two or more thereof may be used in an appropriate combination.

The content of the thickener in a coloring solution of the present invention is preferably 0.01 to 10 mass%, more preferably 0.02 to 8 mass%, even more preferably 0.05 to 5 mass%.

A coloring solution of the present invention may comprise other additives, provided that it does not hinder the effects of the present invention.

Examples of the additives include stabilizers (for example, methoxyphenol, hydroquinone, Topanol A (2,4-dimethyl-6-tert-butylphenol), and a mixture of these (excluding a stabilizer capable of reducing a phase transformation of zirconia)), buffers (for example, acetate, amino buffer, and a mixture of these), preservatives (for example, sorbic acid, benzoic acid, and a mixture of these), and a mixture of these. The additives may be used alone, or two or more thereof may be used in combination.

The content of the additive in a coloring solution of the present invention may be, for example, 0.01 to 10 mass%, 0.05 to 5 mass%, or 0.1 to 3 mass%.

In a coloring solution of the present invention, the coloring component may comprise a colorant that becomes decolored after zirconia is fired. The colorant that becomes decolored after zirconia is fired is not particularly limited, as long as it is decolored after firing zirconia, and can provide a satisfactory color difference before and after firing. The colorant may be, for example, an organic dye.

The organic dye is not particularly limited, as long as it is an organic dye having a chromophore, and that dissolves in the coloring solution. Preferably, the organic dye is an aromatic organic dye, that is, an organic dye containing one or more aromatic groups that may be substituted. More preferably, the organic dye is an aromatic organic dye having an auxochrome, in addition to a chromophore. The chromophore is not particularly limited, as long as it is a group of atoms that confer color by binding to an aromatic ring. Examples include a nitro group, an azo group, a ketimide group (>C=N-), a carbonyl group, a carbon-carbon double bond, a carbon-carbon triple bond, a carbon-nitrogen multiple bond, a thiocarbonyl group, a nitroso group, and an azoxy group. The organic dye may contain one of such groups of atoms alone, or may contain two or more groups of atoms in an appropriate combination. Examples of the auxochrome include a hydroxyl group, an amino group, a carboxyl group, a sulfone group, and a halogen atom. The organic dye may contain one of such auxochromes alone, or may contain two or more auxochromes in an appropriate combination.

The organic dye is preferably a food dye, more preferably a food dye that dissolves in the coloring solution, because the colorant that becomes decolored after firing zirconia must be harmless and nontoxic to humans. Examples of such food dyes include:

organic dyes containing two or more aromatic groups, such as yellow 4 (tartrazine), yellow 5 (sunset yellow FCF), red 2 (amaranth), red 102 (new coccin), blue 1 (brilliant blue FCF), blue 2 (indigo carmine), green 3 (fast green FCF), and red 102 (new coccin);

organic dyes containing fused aromatic groups with a xanthene scaffold (xanthene dye), such as acid red 289, bromopyrogallol red, rhodamine B, rhodamine 6G, rhodamine 6GP, rhodamine 3GO, rhodamine 123, eosin (eosin B, eosin Y), fluorescein, and fluorescein isothiocyanate;

cochineal dyes (carmic acid dyes); and

betalain dyes such as beet red (main components: isobetanin and betanin), betanin, isobetanin, probetanin, and neobetanin.

Preferred are organic dyes containing two or more aromatic groups and having a ketimide group or an azo group as a chromophore, for example, such as yellow 4 (tartrazine), yellow 5 (sunset yellow FCF), red 2 (amaranth), red 102 (new coccin), blue 1 (brilliant blue FCF), green 3 (fast green FCF), and isobetanin. Because the colorant (A) can be changed depending on the content of the stabilizer in a zirconia pre-sintered body to which a coloring solution of the present invention is applied, a certain preferred embodiment is, for example, a zirconia coloring solution in which the colorant (A) is an organic dye containing two or more aromatic groups, and having a ketimide group or an azo group as a chromophore, and a sulfone group as an auxochrome. In the present specification, "aromatic group" includes an aromatic group whose ring structure consists solely of carbon atoms, and a heteroaromatic group whose ring structure contains elements other than carbon (e.g., oxygen, nitrogen). The colorant that becomes decolored after firing zirconia may be used alone, or two or more thereof may be used in an appropriate combination. The colorant that becomes decolored after firing zirconia may produce different color intensities depending on the pH of the coloring solution, and may take different compound structures for different pH values. However, the pH of the zirconia coloring solution is not particularly limited, as long as the present invention can exhibit its effects. A pH value may be selected that provides an appropriate color intensity, according to the type of the colorant that becomes decolored after firing zirconia.

The content of the colorant that becomes decolored after firing zirconia is not particularly limited, as long as the liquid component can produce color. Preferably, the content of the colorant that becomes decolored after firing zirconia is 0.009 to 3.0 mass%, more preferably 0.09 to 1.6 mass%, even more preferably 0.2 to 1.4 mass%, particularly preferably 0.25 to 1.2 mass% relative to the mass of the whole coloring solution.

A certain embodiment is, for example, a coloring solution in which the coloring component is essentially free of a colorant that becomes decolored after firing zirconia. By "essentially free of a colorant that becomes decolored after firing zirconia", it means that the content of the colorant that becomes decolored after firing zirconia is preferably less than 0.009 mass%, more preferably less than 0.001 mass%, even more preferably less than 0.0001 mass% relative to the mass of the whole coloring solution. The content of the colorant that becomes decolored after firing zirconia may be 0 mass%.

With a coloring solution of the present invention, the pink shade needed for dental use can be imparted to a dental ceramic. A dental ceramic after coloring and sintering has chromaticity (L* a* b*) with a* of preferably 4.5 to 15.0, more preferably 4.8 to 14.5, even more preferably 5.0 to 14.0 according to L*a*b*color system. Preferably, b* is -5.0 to 10, more preferably -4.4 to 9, even more preferably -4.0 to 8. Preferably, L* is 65 to 95, more preferably 68 to 92, even more preferably 70 to 90. The method of measurement of chromaticity (L*,a*,b*) is as described in the EXAMPLES section below.

A dental ceramic colored with a coloring solution of the present invention is not particularly limited, as long as it contains ceramic. Examples include those containing, for example, zirconia (zirconium oxide or $ZrO_2$ as it is also called), alumina (aluminum oxide or $Al_2O_3$ as it is also called), feldspar glass, disilicate glass, or porcelain. Preferred as dental ceramics are those containing zirconia and/or alumina, more preferably those containing zirconia as a main component. In the case of a dental ceramic containing zirconia as a main component, the zirconia content is more preferably 65 mass% or more, particularly preferably 75 mass% or more, most preferably 85 mass% or more.

A dental ceramic colored with a coloring solution of the present invention may be an unfired body or a pre-sintered body, provided that it is not sintered. However, in view of penetration of the coloring solution, a zirconia pre-sintered body is preferred in the case of a dental ceramic containing zirconia as a main component.

Another embodiment of the present invention is, for example, a dental ceramic (a colored ceramic pre-sintered body or unfired body) supporting an Er component and a Co component on a surface of the dental ceramic. The content of the Er component and Co component is not particularly limited, as long as the present invention can exhibit its effects. For example, the content of the Er component and Co component can be appropriately adjusted by adjusting the applied amount of a coloring solution of the present invention, according to, for example, the desired color intensity to be obtained after sintering. The Er component and Co component can be adjustably supported in an appropriate fashion not only on the outermost surface but in the surface of a ceramic pre-sintered body or unfired body because the Er component and Co component can penetrate into a ceramic pre-sintered body or unfired body by capillary action through spaces that are in communication with outside, following application of, for example, a coloring solution of the present invention. By "support", it generally means a state of adhesion to a support. In the present invention, "support" refers to a state of adhesion to a ceramic by means of, for example, adsorption.

With a dental ceramic of the present invention, the pink shade needed for dental use can be imparted after firing, without decreasing the strength.

Preferably, the ceramic pre-sintered body or unfired body comprises zirconia as a main component, as noted above. Below is a description of zirconia. In the present invention, a pre-sintered body after coloring is called "colored zirconia pre-sintered body" to distinguish it from "zirconia pre-sintered body", a simplified term used herein to refer to a pre-sintered body before coloring with the coloring solution. A coloring solution of the present invention can also be used to color a zirconia unfired body. In this case, a zirconia sintered body is produced without producing a pre-sintered body. In situations assuming such sintered bodies, the conditions in the descriptions given below in conjunction with zirconia pre-sintered bodies are equally applicable as preferred embodiments of the zirconia unfired body.

The following describes a zirconia pre-sintered body of the present invention. The zirconia pre-sintered body refers to a material containing zirconia ($ZrO_2$: zirconium oxide) as a main component, and in which zirconia has pre-sintered after the material was molded according to the dental product to be produced. For example, "zirconia pre-sintered body" means a block formed while zirconia particles (powder) are not fully sintered. The main component may be 50 mass% or more. The zirconia content in a zirconia pre-sintered body according to the present invention is preferably 60 mass% or more, more preferably 70 mass% or more, even more preferably 80 mass% or more. For example, for applications as dental prostheses or dental implant products, the zirconia pre-sintered body can be prepared by, for example, pre-sintering a disc or a block obtained by press forming a zirconia powder using a known technique. The zirconia pre-sintered body has a density of preferably 2.7 g/cm$^3$ or more. The zirconia pre-sintered body has a density of preferably 4.0 g/cm$^3$ or less, more preferably 3.8 g/cm$^3$ or less, even more preferably 3.6 g/cm$^3$ or less. Molding can be performed with ease when the density is confined in these ranges. The density of the pre-sintered body can be calculated as, for example, a ratio of the mass of the pre-sintered body to the volume of the pre-sintered body. The zirconia pre-sintered body has a three-point flexural strength of preferably 15 to 70 MPa, more preferably 18 to 60 MPa, even more preferably 20 to 50 MPa. The flexural strength can be measured following ISO 6872:2015 except for the specimen size, using a specimen measuring 5 mm in thickness, 10 mm in width, and 50 mm in length. For surface finishing, the specimen surfaces, including chamfered surfaces (45° chamfers at the corners of specimen), are finished longitudinally with #600 sandpaper. The specimen is disposed in such an orientation that its widest face is perpendicular to the vertical direction (loading direction). In the flexure test, measurements are made at a span of 30 mm with a crosshead speed of 0.5 mm/min.

It is preferable that the zirconia in the zirconia pre-sintered body and in a zirconia powder and a molded body of zirconia powder be predominantly monoclinic in crystal system. In the present invention, "being predominantly monoclinic in crystal system" means that the fraction $f_m$ of the monoclinic crystal system of zirconia calculated from the formula (1) below is 50% or more relative to the total amount of all the crystal systems (monoclinic, tetragonal, and cubic) of zirconia. In the zirconia pre-sintered body and in a zirconia powder and a molded body of zirconia powder, the fraction $f_m$ of the monoclinic crystal system of zirconia calculated from the formula (1) below is preferably 55% or more, more preferably 60% or more, even more preferably 70% or more, yet more preferably 75% or more, particularly preferably 80% or more, still more preferably 85% or more, most preferably 90% or more relative to the total amount of the monoclinic, tetragonal, and cubic crystal systems. The fraction $f_m$ of monoclinic crystal system can be calculated from the formula (1) below, using peaks in an X-ray diffraction (XRD) pattern by CuKα radiation. The predominant crystal system possibly contributes to elevating the shrinkage temperature in firing the zirconia pre-sintered body, and reducing the firing time.

In the zirconia pre-sintered body, the peaks for tetragonal and cubic crystal systems may be essentially undetectable. That is, the fraction $f_m$ of the monoclinic crystal system may be 100%.

[Math. 1]

$$f_m(\%) = \frac{I_m(111) + I_m(11-1)}{I_m(111) + I_m(11-1) + I_t(111) + I_c(111)} \times 100 \qquad (1)$$

In formula (1), $I_m(111)$ and $I_m(11-1)$ represent the peak intensities of the (111) plane and (11-1) plane, respectively, of the monoclinic crystal system of zirconia, $I_t(111)$ represents the peak intensity of the (111) plane of the tetragonal crystal system of zirconia, and $I_c(111)$ represents the peak intensity of the (111) plane of the cubic crystal system of zirconia.

Preferably, a zirconia pre-sintered body of the present invention comprises a stabilizer capable of reducing a phase transformation of zirconia. For example, a stabilizer capable of reducing a phase transformation of zirconia is preferably contained in zirconia before pre-sintering.

Examples of the stabilizer capable of reducing a phase transformation of zirconia include oxides such as yttrium oxide ($Y_2O_3$; hereinafter "yttria"), calcium oxide (CaO), magnesium oxide (MgO), yttria, cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide (PreOn), samarium oxide ($Sm_2O_3$), europium oxide ($Eu_2O_3$), and thulium oxide ($Tm_2O_3$). Preferred is yttria. These may be used alone, or two or more thereof may be used in combination. In a certain preferred embodiment of the coloring solution, a dental ceramic to be colored contains zirconia as a main component, and yttria as a stabilizer, and the stabilizer consists essentially of yttria. In such a preferred embodiment, "stabilizer consisting essentially of yttria" means that the content of stabilizers other than yttria is less than 0.1 mol%, preferably 0.05 mol% or less, more preferably 0.01 mol% or less, even more preferably 0.001 mol% or less in total 100 mol% of zirconia and stabilizer. Particularly, because the coloring solution contains an Er component, it is preferable in the foregoing preferred embodiment that the dental ceramic do not contain erbium oxide as a stabilizer because the pink color will be darker than expected when the dental ceramic contains an Er component besides the Er component contained in a coloring solution according to the present invention. However, in such an embodiment, the dental ceramic containing no erbium oxide as a stabilizer may contain a trace amount of erbium oxide as a pigment.

When the zirconia pre-sintered body contains a stabilizer, the stabilizer content is preferably 0.1 to 18 mol%, more preferably 1 to 15 mol%, even more preferably 1.5 to 10 mol% in total 100 mol% of zirconia and stabilizer. A certain preferred embodiment includes a dental ceramic and a coloring solution for coloring same, in which the dental ceramic contains zirconia as a main component, and the yttria content is 1.5 to 10 mol% relative to the total mole of zirconia and yttria. In such a preferred embodiment, the yttria content is preferably 2.0 to 9.0 mol%, more preferably 2.5 to 8.5 mol%, even more preferably 2.8 to 8.0 mol%.

A zirconia pre-sintered body of the present invention may optionally comprise, for example, a colorant (including a pigment, a composite pigment, and a fluorescent agent), alumina ($Al_2O_3$), titanium oxide ($TiO_2$), or silica ($SiO_2$). These components may be used alone, or two or more thereof may be used as a mixture. Examples of the pigment include an oxide of at least one element selected from the group consisting of Ti, V, Cr, Mn, Fe, Co, Ni, Zn, Y, Zr, Sn, Sb, Bi, Ce, Pr, Sm, Eu, Gd, Tb, and Er. Examples of the composite pigment include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$. Examples of the fluorescent agent include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

A typical method of production of a zirconia pre-sintered body of the present invention is as follows. First, granules are prepared that comprise a zirconia raw material containing a stabilizer (preferably, zirconia particles that are predominantly monoclinic in crystal system), and the granules are pressed into a shape such as a block or a disc. Optionally, the molded body is subjected to CIP (Cold Isostatic Pressing). The applied pressure is, for example, 50 to 500 MPa. This is followed by pre-sintering. Pre-sintering can produce a zirconia pre-sintered body by gradually increasing temperature from room temperature to 800 to 1,200°C, and retaining this temperature for about 1 to 6 hours. The resulting zirconia pre-sintered body can then be milled with a known device, according to the final dental product. For example, when the dental product is a dental prosthesis, the zirconia pre-sintered body is milled into a shape of a dental cap with a CAD/CAM or other devices.

The zirconia pre-sintered body may be a commercially available product. Examples of such commercially available products include Noritake KATANA® zirconia (Model: Disc UTML, Disc STML, Disc ML, Disc HT, Disc LT; all manufactured by Kuraray Noritake Dental Inc.).

A method of production of a colored zirconia pre-sintered body of the present invention comprises the step of containing the coloring solution in a zirconia pre-sintered body after milling. The coloring solution may be contained by, for example, being applied to the zirconia pre-sintered body with a brush or the like, dipping the zirconia pre-sintered body in a container containing the coloring solution, or spraying the coloring solution to the zirconia pre-sintered body with a sprayer or the like. Known tools and devices can be used. When a zirconia sintered body is produced directly from an unfired body without the pre-sintering step, the coloring solution may be contained in the zirconia unfired body after milling.

The present invention also encompasses a zirconia sintered body formed from the colored zirconia pre-sintered body. A method of production of the zirconia sintered body comprises the step of firing the colored zirconia pre-sintered body. The firing temperature (highest temperature in firing) can be appropriately selected according to the type of zirconia, and is not particularly limited as long as the Er component and Co component contained in the coloring solution can develop color. However, the firing temperature is preferably 1,350°C or more, more preferably 1,450°C or more, even more preferably 1,500°C or more. The upper limit of firing temperature is not particularly limited, and is, for example, preferably 1,700°C or less. The zirconia sintered body encompasses not only sintered bodies after sintering of molded zirconia particles under ordinary pressure or no applied pressure, but sintered bodies compacted by a high-temperature pressing process such as HIP (Hot Isostatic Pressing).

The stabilizer content in the zirconia sintered body can be measured, for example, by inductively coupled plasma (ICP) emission spectral analysis or X-ray fluorescence analysis.

Preferably, a zirconia sintered body of the present invention has at least one of partially stabilized zirconia and fully stabilized zirconia as the matrix phase. In the zirconia sintered body, the predominant crystalline phase of zirconia is at least one of the tetragonal crystal system and the cubic crystal system. The zirconia sintered body may have both the tetragonal crystal system and the cubic crystal system. Preferably, the zirconia sintered body is essentially free of a monoclinic crystal system. Zirconia that is partially stabilized by addition of a stabilizer is called partially stabilized zirconia (PSZ), whereas zirconia that is fully stabilized is called fully stabilized zirconia.

The present invention encompasses dental products formed from the zirconia sintered body. Examples of the dental products include dental prostheses, orthodontic products, and dental implant products. The dental prostheses can be used as, for example, zirconia inlays, onlays, laminate veneers, or crowns.

In all of the foregoing embodiments, aspects such as the type and content of each component can be changed as appropriate, and changes such as additions and deletions can be made in any of the components. In all of the foregoing embodiments, the composition and property values of the coloring solution can be varied and combined as appropriate.

The present invention encompasses embodiments combining the foregoing features, provided that the present invention can exhibit its effects with such combinations made in various forms within the technical scope of the present invention.


EXAMPLES


The following describes the present invention in greater detail by way of Examples. The present invention, however, is not limited by the following descriptions.


Examples 1 to 17 and Comparative Examples 1 to 3


Coloring solutions were prepared for Examples and Comparative Examples, and their properties were evaluated, as follows. The results are presented in Tables 1 and 2.


Preparation of Coloring Solution


The components shown in Tables 1 and 2 were mixed at ordinary temperature in the amounts shown in Tables 1 and 2 to prepare coloring solutions. The molar concentration of each metallic component in the coloring solutions was measured by inductively coupled plasma (ICP) emission spectral analysis (SPS3500, manufactured by Hitachi High-Tech Science Corporation).


Preparation of Zirconia Pre-Sintered Body


The following describes the preparation of a zirconia pre-sintered body to which the coloring solution is applied.

First, a zirconia powder containing a stabilizer was prepared. A mixture was prepared by adding 9.9 mass% (5.5 mol%) of yttria as stabilizer to 90.1 mass% of a zirconia powder that is predominantly monoclinic in crystal system. The mixture was added to water to prepare a slurry, and the slurry was mixed and pulverized to an average particle diameter of 0.13 $\mu$m or less by wet pulverization with a ball mill. After pulverization, the slurry was dried with a spray dryer, and the resulting powder was fired at 950°C for 2 hours to prepare a powder (primary powder). The average particle diameter can be determined by a laser diffraction scattering method. As a specific example of a laser diffraction scattering method, a 0.2% aqueous solution of sodium hexametaphosphate may be used as a dispersion medium for the measurement of average particle diameter by volume, using a laser diffraction particle size distribution analyzer (SALD-2300, manufactured by Shimadzu Corporation).

Water was added to the primary powder to prepare a slurry, and the slurry was mixed and pulverized to an average particle diameter of 0.13 $\mu$m or less by wet pulverization with a ball mill. After adding a binder to the pulverized slurry, the slurry was dried with a spray dryer to prepare a powder (secondary powder). The secondary powder was used as a raw material powder for the production of the zirconia pre-sintered body below.

The method of production of a zirconia pre-sintered body is as follows. The raw material powder (1.32 g) was filled into a die measuring 19 mm in diameter, and was pressed under a surface pressure of 57.5 kN for 20 seconds with a uniaxial pressing machine (primary press forming). The resulting primary press-molded body was then fired at 1,000°C for 2 hours to prepare a zirconia pre-sintered body.


Measurement of Chromaticity of Sintered Body, and Color Evaluation


The coloring solution prepared was applied to the zirconia pre-sintered body produced, and the zirconia pre-sintered body was fired into a sintered body under the firing conditions shown in Tables 1 and 2. The sintered body was ground into a circular disc measuring 15 mm in diameter and 1.2 mm in thickness, and was measured for chromaticity against a white background according to L*a*b*color system (JIS Z 8781-4:2013 Color Measurements - Part 4: CIE 1976 L*a*b* color space), using a spectrophotometer (Crystaleye CE100-DC/JP, 7-band LED light source) manufactured by Olympus Corporation under this trade name (n = 3). Tables 1 and 2 show the mean values of measured values.

Separately, the sintered body was visually evaluated for its color.

Measurement of Biaxial Flexural Strength of Sintered Body

The coloring solution prepared was applied to the zirconia pre-sintered body produced, and the zirconia pre-sintered body was fired into a sintered body (15 mm in diameter, 1.2 mm in thickness) under the firing conditions shown in Tables 1 and 2. The sintered body was measured for biaxial flexural strength in compliance with JIS T 6526:2012 at a crosshead speed of 0.5 mm/min, using a desktop universal precision tester Autograph (AGS-X) manufactured by Shimadzu Corporation under this trade name (n = 5). Tables 1 and 2 show the mean values of measured values.

Separately, the percentage change of biaxial flexural strength was calculated using the following formula, relative to the zirconia sintered body that was not colored with a coloring solution (Comparative Example 1).

Percentage change of biaxial flexural strength (%) = {(biaxial flexural strength of sintered body fired after application of coloring solution - biaxial flexural strength of uncolored zirconia sintered body)/biaxial flexural strength of uncolored zirconia sintered body} × 100

[Table 1]

| Components (parts by mass) | | EX. 1 | EX. 2 | EX. 3 | EX. 4 | EX. 5 | EX. 6 | EX. 7 | EX. 8 | EX. 9 | EX. 10 |
|---|---|---|---|---|---|---|---|---|---|---|---|
| Er component | Erbium chloride hydrate | 5.00 | 8.00 | 10.0 | 5.00 | 8.00 | 10.0 | 10.0 | 10.0 | 8.00 | 8.00 |
| | Erbium acetate hydrate | 0.00 | 0.00 | 0.00 | 0.00 | 0.00 | 0.00 | 0.00 | 0.00 | 0.00 | 0.00 |
| Co component | Cobalt(II) acetate hydrate | 0.00500 | 0.00500 | 0.00500 | 0.00500 | 0.00500 | 0.00500 | 0.0300 | 0.00100 | 0.00500 | 0.00500 |
| | Cobalt(II) chloride hydrate | 0.00 | 0.00 | 0.00 | 0.00 | 0.00 | 0.00 | 0.00 | 0.00 | 0.00 | 0.00 |
| Al component | Aluminum chloride hydrate | 0.00 | 0.00 | 0.00 | 0.100 | 0.100 | 0.100 | 0.100 | 0.100 | 0.500 | 0.0100 |
| | Aluminum nitrate hydrate | 0.00 | 0.00 | 0.00 | 0.00 | 0.00 | 0.00 | 0.00 | 0.00 | 0.00 | 0.00 |
| Solvent | Purified water | 95.0 | 92.0 | 90.0 | 94.9 | 91.9 | 89.9 | 89.9 | 89.9 | 91.5 | 92.0 |
| | Ethanol | 0.00 | 0.00 | 0.00 | 0.00 | 0.00 | 0.00 | 0.00 | 0.00 | 0.00 | 0.00 |
| | Ethylene glycol | 0.00 | 0.00 | 0.00 | 0.00 | 0.00 | 0.00 | 0.00 | 0.00 | 0.00 | 0.00 |
| Zr component | Zirconium(IV) chloride oxide hydrate | 0.00 | 0.00 | 0.00 | 0.00 | 0.00 | 0.00 | 0.00 | 0.00 | 0.00 | 0.00 |
| Complexing agent | N,N-Di(2-hydroxyethyl) glycine | 0.00 | 0.00 | 0.00 | 0.00 | 0.00 | 0.00 | 0.00 | 0.00 | 0.00 | 0.00 |
| Er ion concentration [mmol/L] | | 136 | 222 | 282 | 136 | 222 | 282 | 282 | 282 | 223 | 222 |
| Co ion concentration [mmol/L] | | 0.208 | 0.213 | 0.216 | 0.208 | 0.213 | 0.216 | 1.30 | 0.0433 | 0.214 | 0.213 |
| Al ion concentration [mmol/L] | | 0.00 | 0.00 | 0.00 | 4.30 | 4.40 | 4.46 | 4.47 | 4.46 | 22.0 | 0.439 |
| Zr ion concentration [mol/L] | | 0.00 | 0.00 | 0.00 | 0.00 | 0.00 | 0.00 | 0.00 | 0.00 | 0.00 | 0.00 |
| Firing temperature [°C] *1 | | 1550 | 1550 | 1550 | 1550 | 1550 | 1550 | 1550 | 1550 | 1550 | 1550 |
| Chromaticity after firing | L* | 87.28 | 85.75 | 85.42 | 87.10 | 86.69 | 86.04 | 83.67 | 85.31 | 85.63 | 85.55 |
| | a* | 5.35 | 7.96 | 9.58 | 6.41 | 8.04 | 9.69 | 10.78 | 10.18 | 9.37 | 8.48 |
| | b* | 2.78 | 0.44 | 0.02 | 1.32 | 1.03 | -0.03 | -1.27 | -0.20 | 0.02 | 0.25 |
| Visual color evaluation after firing | | Pink | Pink | Pink | Pink | Pink | Pink | Pink | Pink | Pink | Pink |
| Biaxial flexural strength of zirconia sintered body [MPa] | | 640 | 645 | 641 | 658 | 655 | 642 | 645 | 661 | 690 | 687 |

13

(continued)

| Components (parts by mass) | EX. 1 | EX. 2 | EX. 3 | EX. 4 | EX. 5 | EX. 6 | EX. 7 | EX. 8 | EX. 9 | EX. 10 |
|---|---|---|---|---|---|---|---|---|---|---|
| Percentage change of biaxial flexural strength (%) | -1.1 | -0.3 | -0.8 | 1.7 | 1.2 | -0.8 | -0.3 | 2.1 | 6.6 | 6.2 |
| *1: In the Table, "Firing temperature" means the highest temperature in firing. Conditions other than firing temperature: Temperature increase from room temperature to 1,550°C at 10°C /min; retention at 1,550°C for 2 hours; temperature decrease at -10°C/min and left to cool from 300°C to room temperature. | | | | | | | | | | |

[Table 2]

| Components (parts by mass) | | EX. 11 | EX. 12 | EX. 13 | EX. 14 | EX. 15 | EX. 16 | EX. 17 | Com. Ex. 1 | Com. Ex. 2 | Com. Ex. 3 |
|---|---|---|---|---|---|---|---|---|---|---|---|
| Er component | Erbium chloride hydrate | 0.00 | 8.00 | 8.00 | 8.00 | 8.00 | 8.00 | 8.00 | | 15.0 | 0.00 |
| | Erbium acetate hydrate | 8.70 | 0.0 | 0.0 | 0.0 | 0.00 | 0.00 | 0.00 | | 0.00 | 0.00 |
| Co component | Cobalt(II) acetate hydrate | 0.00 | 0.00500 | 0.00500 | 0.00500 | 0.00500 | 0.00500 | 0.00500 | | 0.00 | 0.500 |
| | Cobalt(II) chloride hydrate | 0.00480 | 0.0 | 0.0 | 0.0 | 0.00 | 0.00 | 0.00 | | 0.00 | 0.00 |
| Al component | Aluminum chloride hydrate | 0.00 | 0.100 | 0.100 | 0.100 | 0.100 | 0.100 | 0.100 | No application of coloring solution | 0.00 | 0.00 |
| | Aluminum nitrate hydrate | 0.150 | 0.0 | 0.0 | 0.0 | 0.00 | 0.00 | 0.00 | | 0.00 | 0.00 |
| Solvent | Purified water | 91.1 | 88.9 | 81.9 | 73.4 | 0.00 | 0.00 | 91.8 | | 85.0 | 99.5 |
| | Ethanol | 0.00 | 0.00 | 0.00 | 0.00 | 91.9 | 0.00 | 0.00 | | 0.00 | 0.00 |
| | Ethylene glycol | 0.00 | 0.00 | 0.00 | 0.00 | 0.00 | 91.9 | 0.00 | | 0.00 | 0.00 |
| Zr component | Zirconium(IV) chloride oxide hydrate | 0.00 | 3.00 | 10.0 | 18.5 | 0.00 | 0.00 | 0.00 | | 0.00 | 0.00 |
| Complexing agent | N,N-Di(2-hydroxyethyl)glycine | 0.00 | 0.00 | 0.00 | 0.00 | 0.00 | 0.00 | 0.100 | | 0.00 | 0.00 |
| Er ion concentration [mmol/L] | | 225 | 226 | 236 | 250 | 222 | 222 | 223 | | 440 | 0.00 |
| Co ion concentration [mmol/L] | | 0.218 | 0.217 | 0.226 | 0.239 | 0.213 | 0.213 | 0.213 | | 0.00 | 20.1 |
| Al ion concentration [mmol/L] | | 4.31 | 4.48 | 4.67 | 4.93 | 4.40 | 4.40 | 4.40 | | 0.00 | 0.00 |
| Zr ion concentration [mol/L] | | 0.00 | 0.101 | 0.350 | 0.684 | 0.00 | 0.00 | 0.00 | | 0.00 | 0.00 |
| Firing temperature [°C] *1 | | 1550 | 1550 | 1550 | 1550 | 1550 | 1550 | 1550 | 1550 | 1550 | 1550 |

(continued)

| Components (parts by mass) | | EX. 11 | EX. 12 | EX. 13 | EX. 14 | EX. 15 | EX. 16 | EX. 17 | Com. Ex. 1 | Com. Ex. 2 | Com. Ex. 3 |
|---|---|---|---|---|---|---|---|---|---|---|---|
| Chromaticity after firing | L* | 86.79 | 85.55 | 85.42 | 85.31 | 86.78 | 86.51 | 86.82 | 90.74 | 84.99 | 63.81 |
| | a* | 8.14 | 8.48 | 8.52 | 8.53 | 8.15 | 8.01 | 8.14 | -2.13 | 13.85 | 4.18 |
| | b* | 1.05 | 0.25 | 0.18 | 0.21 | 1.08 | 1.05 | 1.09 | 5.36 | -0.56 | -8.88 |
| Visual color evaluation after firing | | Pink | Pink | Pink | Pink | Pink | Pink | Pink | White | Pink | Blue-purple |
| Biaxial flexural strength of zirconia sintered body [MPa] | | 650 | 689 | 695 | 702 | 655 | 652 | 659 | 647 | 415 | 642 |
| Percentage change of biaxial flexural strength (%) | | 0.5 | 6.5 | 7.4 | 8.5 | 1.2 | 0.8 | | - | -35.9 | -0.8 |
| *1: In the Table, "Firing temperature" means the highest temperature in firing. Conditions other than firing temperature: Temperature increase from room temperature to 1,550°C at 10°C /min; retention at 1,550°C for 2 hours; temperature decrease at -10°C/min and left to cool from 300°C to room temperature. | | | | | | | | | | | |

As shown in Table 2, the color was white, and a pink color was not observed in Comparative Example 1 in which a coloring solution was not applied. The biaxial flexural strength showed a 35.9% decrease in Comparative Example 2 in which a coloring solution containing only the Er component was used for coloring, compared to Comparative Example 1 in which a coloring solution was not applied. Comparative Example 3 in which a coloring solution containing only the Co component was used for coloring showed a blue-purple color, and did not produce a pink color. In contrast, in Examples 1 to 17, it was possible to produce a pink color while reducing a decrease of zirconia strength, as shown in Tables 1 and 2.

INDUSTRIAL APPLICABILITY

With a coloring solution of the present invention, the pink shade needed for dental use can be imparted while reducing a decrease in the strength of a dental ceramic. This enables a coloring solution of the present invention to be suitably used as a dental ceramic coloring solution. A dental ceramic coloring solution of the present invention is useful given an expected increase in the use of dental ceramic coloring solutions, particularly in response to the continuously growing demand for ceramic dental caps and the associated increase in individual demand for better aesthetics. A coloring solution of the present invention can be suitably used for prostheses that include a gingival part. A prosthesis including a gingival part may be an implant's upper structure used beneath the gingival margin, or a large dental prosthesis called ALL ON 4 (a procedure with balanced placement of four implants in the bone), which enables prosthetic reproduction of teeth, including the gingival area.

**Claims**

1. A coloring solution for coloring a dental ceramic, comprising a coloring component and a solvent, wherein the coloring component comprises an Er component and a Co component.

2. The coloring solution according to claim 1, wherein a dental ceramic after coloring and sintering has an a* of 4.5 to 15.0, and a b* of -5.0 to 10 in (L*,a*,b*) according to L*a*b*color system.

3. The coloring solution according to claim 1 or 2, wherein L* in (L*,a*,b*) according to L*a*b*color system is 65 to 95.

4. The coloring solution according to any one of claims 1 to 3, wherein the coloring component further comprises an Al component.

5. The coloring solution according to any one of claims 1 to 4, wherein the Er component is an ion or a complex.

6. The coloring solution according to any one of claims 1 to 5, wherein the Er component is a component derived from at least one selected from the group consisting of erbium chloride hydrate, erbium perchlorate hydrate, erbium nitrate hydrate, erbium oxalate hydrate, and erbium acetate hydrate.

7. The coloring solution according to any one of claims 1 to 6, wherein the Co component is an ion or a complex.

8. The coloring solution according to any one of claims 1 to 6, wherein the Co component is a component derived from at least one selected from the group consisting of cobalt(II) chloride hydrate, cobalt(II) perchlorate hydrate, cobalt(II) fluoride hydrate, cobalt(II) nitrate hydrate, cobalt(II) oxalate hydrate, and cobalt(II) acetate hydrate.

9. The coloring solution according to any one of claims 1 to 8, wherein the content of the Er component is 110 to 310 mmol/L in terms of an Er ion.

10. The coloring solution according to any one of claims 1 to 9, wherein the content of the Co component is 0.0340 to 1.70 mmol/L in terms of a Co ion.

11. The coloring solution according to any one of claims 1 to 10, wherein the solvent comprises water and/or an organic solvent.

12. The coloring solution according to claim 11, wherein the organic solvent comprises at least one selected from the group consisting of an alcohol, a glycol, a triol, and a ketone.

13. The coloring solution according to any one of claims 1 to 12, wherein the dental ceramic comprises zirconia as a main component.

14. The coloring solution according to claim 13, wherein the dental ceramic further comprises yttria.

15. The coloring solution according to claim 14, wherein the content of the yttria is 1.5 to 10 mol% relative to a total mole of the zirconia and the yttria.

16. A dental ceramic supporting an Er component and a Co component on a surface of the dental ceramic.

17. The dental ceramic according to claim 16, which additionally supports an Al component on a surface of the dental ceramic.

18. The dental ceramic according to claim 16 or 17, wherein the Er component is an ion or a complex.

19. The dental ceramic according to any one of claims 16 to 18, wherein the Co component is an ion or a complex.

20. The dental ceramic according to any one of claims 16 to 19, wherein the dental ceramic comprises zirconia as a main component.

21. The dental ceramic according to claim 20, wherein the dental ceramic further comprises yttria.

22. The dental ceramic according to claim 21, wherein the content of the yttria is 1.5 to 10 mol% relative to a total mole of the zirconia and the yttria.

# EP 4 223 271 A1

| INTERNATIONAL SEARCH REPORT | | International application No. |
|---|---|---|
| | | **PCT/JP2021/036505** |

**A. CLASSIFICATION OF SUBJECT MATTER**

*A61K 6/78*(2020.01)i; *A61C 13/00*(2006.01)i; *A61C 13/083*(2006.01)i; *A61K 6/818*(2020.01)i; *A61K 6/822*(2020.01)i
FI: A61K6/78; A61C13/00 A; A61C13/083; A61K6/818; A61K6/822

According to International Patent Classification (IPC) or to both national classification and IPC

**B. FIELDS SEARCHED**

Minimum documentation searched (classification system followed by classification symbols)

A61K6/78; A61C13/00; A61C13/083; A61K6/818; A61K6/822

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | US 2019/0233340 A1 (JAMES R. GLIDEWELL DENTAL CERAMICS, INC.) 01 August 2019 (2019-08-01) | 1, 5-16, 18-22 |
| Y | claims, paragraphs [0020], [0046], [0050], examples | 2-4, 17 |
| Y | JP 2017-148374 A (KYOCERA CORP) 31 August 2017 (2017-08-31) paragraphs [0010]-[0012] | 2, 3 |
| Y | JP 2014-522365 A (IVOCLAR VIVADENT AG) 04 September 2014 (2014-09-04) paragraph [0038] | 4, 17 |

☐ Further documents are listed in the continuation of Box C.    ☑ See patent family annex.

| | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/036505**

| Patent document cited in search report | | | Publication date (day/month/year) | Patent family member(s) | | | Publication date (day/month/year) |
|---|---|---|---|---|---|---|---|
| US | 2019/0233340 | A1 | 01 August 2019 | (Family: none) | | | |
| JP | 2017-148374 | A | 31 August 2017 | (Family: none) | | | |
| JP | 2014-522365 | A | 04 September 2014 | US paragraph [0037] CN KR | 2014/0135200 103764598 10-2014-0063526 | A1 A A | |

Form PCT/ISA/210 (patent family annex) (January 2015)

**EP 4 223 271 A1**

## REFERENCES CITED IN THE DESCRIPTION

**Patent documents cited in the description**

- JP 2010534245 T